# EUROPEAN PATENT SPECIFICATION

(11) **EP 2 192 189 B1**
(45) Date of publication and mention of the grant of the patent: **22.08.2018**
(21) Application number: 08827710.8
(22) Date of filing: 22.08.2008
(51) Int. Cl.: C12P 7/46, C12N 15/09

(54) **METHOD FOR PRODUCTION OF SUCCINIC ACID AT HIGH TEMPERATURES**
VERFAHREN ZUR HERSTELLUNG VON BERNSTEINSÄURE BEI HOHEN TEMPERATUREN
PROCÉDÉ DE FABRICATION D'ACIDE SUCCINIQUE À HAUTES TEMPERATURES

(30) Priority: 23.08.2007 JP 2007217277
(43) Date of publication of application: 02.06.2010
(73) Proprietor: Mitsubishi Chemical Corporation, Tokyo 100-8251 (JP)
(72) Inventor: MURASE, Makoto, Tokyo 100-8251 (JP); YONEKURA, Madoka, Tokyo 100-8251 (JP); KIDO, Daisuke, Tokyo 100-8251 (JP); AOYAMA, Ryusuke, Tokyo 100-8251 (JP); YUNOMURA, Shuichi, Tokyo 100-8251 (JP); KOIKE, Sanae, Tokyo 100-8251 (JP)
(74) Representative: Hoffmann Eitle
(86) International application number: PCT/JP2008/065030
(87) International publication number: WO 2009/025363

(56) References cited:
- EP-A1- 1 672 067
- EP-A1- 1 672 077
- WO-A1-2005/010182
- WO-A1-2005/021770
- JP-A- 2003 235 593
- JP-A- 2007 125 030
- JP-A- 2008 067 623
- JP-A- 2008 067 624
- DATABASE WPI Week 200409 Thomson Scientific, London, GB; AN 2004-084946 XP002701617, & JP 2003 235593 A (MITSUBISHI CHEM CORP) 26 August 2003 (2003-08-26)

## Description

### Technical Field

The present invention relates to a method for producing succinic acid using a microorganism.

### Background Art

Methods for producing useful substances using genetically engineered coryneform bacteria have been known (see Patent Literatures 1 and 2). In the case of culturing a coryneform bacterium to produce succinic acid, a method in which bacterial cells are firstly obtained by growing the bacterium under an aerobic condition, and then used as non-growing bacterial cells to allow to react with an organic raw material is often employed. In this case, generally, proliferation of the bacterium is carried out in a range between 25°C and 35°C while the reaction with the organic raw material is carried out in a range between 30°C and 37°C.

In Patent Literature 3, a relationship between growth temperature and reaction temperature, and succinic acid productivity is analyzed for a lactate dehydrogenase-deficient strain of coryneform bacteria. It is shown that the succinic acid productivity can be increased by carrying out a succinic acid production reaction at a temperature which is 2 to 10°C higher than the optimal growth temperature (growth temperature). In the Example 2 of Patent Literature 3, an example in which a succinic acid production reaction was carried out at 40°C using bacterial cells obtained by growing at 30°C. In this case, time to consume saccharides is proven to be slightly longer, when compared with that in Example 1 in which a succinic acid production reaction was carried out at 37°C using bacterial cells obtained by culturing at 30°C.

Also, in Patent Literature 2, a method for producing succinic acid using a coryneform bacterium which has been modified so as to enhance pyruvate carboxylase is known. Yet, reaction temperature and succinic acid productivity have not examined in detail for such a strain.
Patent Literature 1: JP 11-206385 A
Patent Literature 2: JP 11-196888 A
Patent Literature 3: JP 2003-235593 A

### Disclosure of Invention

An object of the present invention is to improve the production efficiency in a method for producing succinic acid using a microorganism.

To solve the above-mentioned problem, the present inventors intensively studied to discover that, in a coryneform bacterium which is modified such that a pyruvate carboxylase activity is enhanced, when a reaction temperature is in a range between 39 and 43°C, the succinic acid productivity improves, therefore completing the present invention.

That is, according to the present invention, the following embodiments are provided.
1. A method for producing succinic acid, said method comprising:
   allowing bacterial cells obtained by culturing a bacterium selected from *Brevibacterium flavum, Brevibacterium lactofermentum* and *Corynebacterium glutamicum,* or a treated product thereof selected from immobilized bacterial cells, a homogenate, centrifuged supernatant thereof, or a fraction, to act on an organic raw material in a reaction solution containing said organic raw material to thereby generate succinic acid, and
   collecting said succinic acid;
   wherein said bacterium has been modified such that pyruvate carboxylase activity is increased by 1.5 times or more per unit weight of bacterial cells, as compared with an unmodified strain, and said bacterial cells obtained by culturing said bacterium or said treated product thereof are allowed to act on said organic raw material at 39 to 43°C.
2. The method according to claim 1, wherein said bacterium has been further modified such that lactate dehydrogenase activity is decreased as compared with an unmodified strain.
3. The method according to claim 1 or 2, wherein said organic raw material is glucose or sucrose.
4. The method according to any one of claims 1 to 3, wherein said reaction solution contains a carbonate ion, a bicarbonate ion, or carbon dioxide gas.
5. The method according to any one of claims 1 to 4, wherein said organic raw material is allowed to act under anaerobic atmosphere.
6. A method for producing a succinic acid-containing polymer, comprising:
   a step of producing succinic acid by the method according to any one of claims 1 to 5; and
   a step of carrying out a polymerization reaction using said succinic acid obtained in the previous step as a raw material.

### Brief Description of the Drawings

[Figure 1] Figure 1 shows a procedure for constructing the plasmid pMJPC17.2. An underlined number represents a primer composed of the sequence of the corresponding SEQ ID NO.
[Figure 2] Figure 2 shows relationships between the reaction temperature and the relative cell specific rate in the MJ233/PC-4/ΔLDH strain and the MJ233/ΔLDH strain.
[Figure 3] Figure 3 shows a relationship between the reaction temperature and the concentration of accumulated succinic acid 6 hours after the beginning of the reaction in the MJ233/PC-4/ΔLDH strain.

### Description of Embodiments

Embodiments of the present invention will be described in detail below.

### 1. Microorganisms used in the present invention

The microorganism used in the method according to the present invention is a bacterium which is selected from *Brevibacterium flavum, Brevibacterium lactofermentum* and *Corynebacterium glutamicum,* and has been modified such that pyruvate carboxylase activity is enhanced as compared with an unmodified strain.

In addition, *Brevibacterium flavum, Brevibacterium lactofermentum* and *Corynebacterium glutamicum* are very closely related each other and have similar characteristics. In the current taxonomy, they are, in some cases, classified into the same species.

Particularly preferred examples of a parent strain of the microorganism used in the present invention include *Brevibacterium flavum* MJ-233 (FERM BP-1497), *Brevibacterium flavum* MJ-233 AB-41 (FERM BP-1498), *Corynebacterium glutamicum* ATCC31831 and *Brevibacterium lactofermentum* ATCC13869.

Since *Brevibacterium flavum* is, in some cases, classified into *Corynebacterium glutamicum* (Lielbl, W., Ehrmann, M., Ludwig, W. and Schleifer, K. H., International Journal of Systematic Bacteriology, 1991, vol. 41, p2SS-260), in the present invention, the *Brevibacterium flavum* MJ-233 strain and its mutant strain, the MJ-233 AB-41 strain, shall be the same strain as the *Corynebacterium glutamicum* MJ-233 strain and MJ-233 AB-41 strain, respectively.

The *Brevibacterium flavum* MJ-233 had been deposited with the Fermentation Research Institute, the Agency of Industrial Science and Technology, the Ministry of International Trade and Industry (currently, International Patent Organism Depositary, National Institute of Advanced Industrial Science and Technology, AIST, an independent administrative institution) (Tsukuba Central 6, 1-1-1 Higashi, Tsukuba, Ibaraki, 305-8566 Japan) under accession No. FERM P-3068, on April 28, 1975; was transferred to the international deposition in accordance with the Budapest Treaty, on May 1, 1981, and is deposited under accession No. FERM BP-1497.

A bacterium which has been modified such that the activity of pyruvate carboxylase (hereinafter, also referred to as PC) is enhanced can be constructed in the same manner as described in, for example, JP 11-196888 A, in which the pc gene is highly expressed in a host bacterium using a plasmid. Also, the gene may be incorporated by homologous recombination, or expression of the pc gene may be enhanced by promoter replacement. Transformation can be carried out by, for example, the electric pulse method (Res. Microbiol., Vol.144, p.181-185, 1993) or the like.

The phrase "PC activity is enhanced" means that the PC activity increases by 1.5 times or more, more preferably by 3 times or more per unit weight of bacterial cells, as compared with an unmodified strain such as a wild type strain or a parent strain. Enhancement of the PC activity can be confirmed by measuring the PC activity using a known method (the method of Magasanik [J. Bacteriol., 158, 55-62, (1984)]).

As a pc gene, for example, the pc gene derived from *Corynebacterium glutamicum* (Peters-Wendisch, P.G. et al., Microbiology, vol.144 (1998) p915-927) can be used.

Further, the pc gene derived from coryneform bacteria other than *Corynebacterium glutamicum,* other microorganisms, or animals and plants can be used. In particular, the sequences of the pc genes derived from the following microorganisms or animals and plants have been already known (references are shown below), and the pc genes can be obtained by hybridization or by amplification of the ORF segments thereof by PCR, in the same manner as described above.
Human [Biochem. Biophys. Res. Comm., 202, 1009-1014, (1994)]
Mouse [Proc. Natl. Acad. Sci .USA., 90, 1766-1779, (1993)]
Rat [GENE, 165, 331-332, (1995)]
Yeast; *Saccharomyces cerevisiae*
   [Mol. Gen. Genet., 229, 307-315, (1991)]
   *Schizosaccharomyces pombe*
   [DDBJ Accession No.; D78170]
*Bacillus stearothermophilus*
   [GENE, 191, 47-50, (1997)]
*Rhizobium etli*
   [J. Bacteriol., 178, 5960-5970, (1996)]

A recombinant plasmid capable of high expression of the pc gene in a host bacterium can be obtained, by introducing a DNA fragment containing the pc gene described above into an appropriate plasmid, for example, a plasmid vector containing at least a gene controlling the replication and proliferation function of the plasmid in the host bacterium. Here, as for the above-mentioned recombinant plasmid, any promoter may be suitable for the expression of the pc gene as long as the promoter consists of a base sequence for initiating the transcription of the pc gene. Examples thereof include a tac promoter, a trc promoter and a TZ4 promoter.

Specific examples of the plasmid usable for introducing a gene into a coryneform bacterium include the plasmid pCRY30 described in JP 3-210184 A; the plasmids pCRY21, pCRY2KE, pCRY2KX, pCRY31, pCRY3KE and pCRY3KX described in JP 2-72876 A and US 5,185,262; the plasmids pCRY2 and pCRY3 described in JP 1-191686 A; pAM330 described in JP 58-67679 A; pHM1519 described in JP 58-77895 A; pAJ655, pAJ611 and pAJ1844 described in JP 58-192900 A; pCG1 described in JP 57-134500 A; pCG2 described in JP 58-35197 A; pCG4 and pCG11 described in JP 57-183799 A. Among them, as a plasmid vector used in a host-vector system for a coryneform bacterium, those having a gene controlling the replication and proliferation function in the coryneform bacterium as well as a gene controlling the function of stabilizing the plasmid in the coryneform bacterium are preferred. For instance, the plasmids such as pCRY30, pCRY21, pCRY2KE, pCRY2KX, pCRY31, pCRY3KE or pCRY3KX can be preferably used.

The bacterium according to the present invention may be a bacterium which has been modified such that a lactate dehydrogenase (also referred to as LDH) activity is decreased, in addition to the enhancement of the PC activity described above.

The bacterium which has been modified such that the LDH activity is decreased may be constructed by disrupting the LDH gene on the chromosome using, for example, a method of homologous recombination described in JP 11-206385 A or a method using a *sacB* gene (Schafer, A. et al., Gene 145 (1994) 69-73). Here, the phrase "LDH activity is decreased" means that the LDH activity is decreased, as compared with an unmodified strain. The LDH activity may be completely lost. The decrease in the LDH activity can be confirmed by measuring the LDH activity using a known method (L. Kanarek and R.L. Hill, J. Biol. Chem. 239, 4202 (1964)).

Further, the bacterium according to the present invention may be a bacterium which has been modified such that activities of one or more types of enzymes selected from the group consisting of acetate kinase (hereinafter, also referred to as ACK), phosphotransacetylase (hereinafter, also referred to as PTA), pyruvate oxidase (hereinafter, also referred to as POXB) and acetyl-CoA hydrolase (hereinafter, also referred to as ACH) are decreased, in addition to the enhancement of the PC activity, or the enhancement of the PC activity and decrease of the LDH described above.

Only either one of PTA or ACK may be subjected to activity decrease. Yet, in order to efficiently decrease the by-production of acetic acid, it is more preferred to decrease both activities.

The term "PTA activity" refers to an activity catalyzing a reaction to transfer phosphate to acetyl CoA to generate acetyl phosphate. The phrase "modified such that the PTA activity is decreased" means that the PTA activity is decreased as compared with an unmodified strain such as a wild type strain. The PTA activity is preferably decreased to not more than 30%, more preferably not more than 10% per unit weight of bacterial cells, as compared with the unmodified strain. Also, the PTA activity may be completely lost. The decrease in the PTA activity can be confirmed by measuring the PTA activity by the method of Klotzsch *et al*. (Klotzsch, H. R., Meth. Enzymol. 12, 381-386(1969)).

The term "ACK activity" refers to an activity catalyzing a reaction to generate acetic acid from acetyl phosphate and ADP. The phrase, "modified such that ACK activity is decreased" means that the ACK activity is decreased as compared with an unmodified strain such as a wild type strain. The ACK activity is preferably decreased to not more than 30%, more preferably not more than 10% per unit weight of bacterial cells, as compared with the unmodified strain. Also, the ACK activity may be completely lost. The decrease in the ACK activity can be confirmed by measuring the ACK activity by the method of Ramponi *et al*. (Ramponi G., Meth. Enzymol. 42,409-426(1975)).

In *Corynebacterium glutamicum* (including those classified in *Brevibacterium flavum*), as describe in Microbiology 1999 Feb; 145 (Pt 2):503-13, both enzymes are encoded by the *pta-ack* operon (GenBank Accession No. X89084). Thus, when the pta gene is disrupted, the activities of both enzymes, PTA and ACK, can be decreased.

The decrease in the activities of PTA and ACK can be attained by disrupting these genes in accordance with a known method, for example, a method using homologous recombination and a method using the *sacB* gene (Schafer, A. et al. Gene 145 (1994) 69-73). Specifically, it can be carried out in accordance with a method disclosed in JP 2006-000091 A. As the *pta* gene and the *ack* gene, besides the above-mentioned gene having the base sequence of GenBank Accession No. X89084, a gene having a homology enough for homologous recombination to occur with the *pta* gene and the *ack* gene on the host chromosome can be used. The term "homology enough for homologous recombination to occur" herein refers to preferably not less than 80% homology, more preferably not less than 90% homology, particularly preferably not less than 95% homology. In addition, the homologous recombination may occur between DNAs each other, as long as the DNAs can hybridize with the above-mentioned gene under stringent conditions.

The term "POXB activity" refers to an activity catalyzing a reaction to generate acetic acid from pyruvic acid and water. The phrase "modified such that the POXB activity is decreased" means that the POXB activity is decreased as compared with an unmodified strain such as a wild type strain. The POXB activity is preferably decreased to not more than 30%, more preferably not more than 10% per unit weight of bacterial cells, as compared with the unmodified strain. The term "decrease" includes a case where the activity is completely lost. The decrease in the POXB activity can be confirmed by measuring the activity by the method of Chang *et al.* (Chang Y. and Cronan J. E. JR, J. Bacteriol. 151, 1279-1289 (1982)).

The decrease in the POXB activity can be attained by disrupting the *poxB* gene in accordance with a known method, for example, a method using homologous recombination and a method using the *sacB* gene (Schafer, A. et al. Gene 145 (1994) 69-73). Specifically, it can be carried out in accordance with a method disclosed in WO2005/113745. An example of the *poxB* gene includes a gene having the base sequence of GenBank Accession No.Cgl2610 (a complement strand of the 2776766th to 2778505th bases of GenBank Accession No. BA000036). A gene homologous to the above sequence can be used as long as it has a homology enough for homologous recombination to occur with the *poxB* gene on the chromosome of the host bacterium. The term "homology enough for homologous recombination to occur" herein refers to preferably not less than 80% homology, more preferably not less than 90% homology, particularly preferably not less than 95% homology. In addition, the homologous recombination may occur between DNAs each other, as long as the DNAs can hybridize with the above-mentioned gene under stringent conditions.

The term "ACH activity" refers to an activity catalyzing a reaction to generate acetic acid from acetyl CoA and water. The phrase "modified such that the ACH activity is decreased" means that the ACH activity is decreased as compared with an unmodified strain such as a wild type strain. The ACH activity is preferably decreased to not more than 30%, more preferably not more than 10% per unit weight of bacterial cells, as compared with the unmodified strain. In addition, the term "decrease" includes a case where the activity is completely lost. The ACH activity can be measured by the method of Gergely, J., *et al*. (Gergely, J., Hele, P. & Ramkrishnan, C.V. (1952) J. Biol. Chem. 198 p323-334).

The decrease in the ACH activity can be attained by disrupting the *ach* gene in accordance with a known method, for example, a method using homologous recombination and a method using the *sacB* gene (Schafer, A. et al. Gene 145 (1994) 69-73). Specifically, it can be carried out in accordance with a method disclosed in WO2005/113744. An example of the *ach* gene includes a gene having the base sequence of GenBank Accession No. Cgl2569 (a complement strand of the 2729376th to 2730917th bases of GenBank Accession No. BA000036). A gene homologous to the above sequence can be used as long as it has a homology enough for homologous recombination to occur with the *ach* gene on the chromosome of the host bacterium. The term "homology enough for homologous recombination to occur" herein refers to preferably not less than 80% homology, more preferably not less than 90% homology, particularly preferably not less than 95% homology. In addition, the homologous recombination may occur between DNAs each other, as long as the DNAs can hybridize with the above-mentioned gene under stringent conditions.

The bacterium used in the present invention may be a bacterium obtained by combining two or more types of the above-mentioned modifications in addition to the above-mentioned enhancement of the PC activity, or the enhancement of the PC activity and the decrease in the LDH activity.

### 2. Method for producing succinic acid

The method for producing succinic acid according to the present invention is a method for producing succinic acid comprising allowing bacterial cells obtained by culturing the above-mentioned bacterium or a treated product thereof as defined in the claims to act on an organic raw material in a reaction solution containing the organic raw material to thereby generate succinic acid, and collecting this succinic acid.

In using the above-mentioned bacterium for the production of succinic acid, one obtained by slant culture on a solid medium such as an agar plate may be directly used for a reaction. Yet, it is preferred to use one obtained by culturing the above-mentioned bacterium in a liquid medium (seed culture) in advance. As a medium for the seed culture, a medium which is usually used for culturing a bacterium can be used. For instance, a common medium composed of inorganic salts such as ammonium sulfate, potassium phosphate or magnesium sulfate, which medium is supplemented with natural nutrients such as meat extract, yeast extract or peptone can be used. It is preferred that the bacterial cells after the seed culture be used for the production reaction of succinic acid after collected by centrifugation, membrane separation or the like. Succinic acid may be produced by allowing the bacterium obtained by the seed culture to react with an organic raw material while the bacterium is growing in a medium containing the organic raw material. Or, succinic acid may also be produced by allowing the bacterial cells obtained by growing in advance to react with an organic raw material in a reaction solution containing the organic raw material.

In the present invention, a treated product of bacterial cells of a bacterium can be used. The treated product of bacterial cells include immobilized bacterial cells which were prepared by immobilizing the bacterial cells with acrylamide, carrageenan or the like, a homogenate obtained by homogenizing the bacterial cells, centrifuged supernatant thereof, or a fraction obtained by partially purifying the supernatant with ammonium sulfate treatment or the like.

The organic raw material used in the production method according to the present invention is not limited as long as it is a carbon source which the present bacterium can assimilate to generate succinic acid. Typically, fermentable carbohydrates including carbohydrates such as galactose, lactose, glucose, fructose, glycerol, sucrose, saccharose, starch or cellulose; and polyalcohols such as glycerin, mannitol, xylitol or ribitol are used. Among these, glucose or sucrose is preferred.

A saccharified starch solution, molasses or the like, which contains the above-mentioned fermentable carbohydrate, can also be used. These fermentable carbohydrates may be used either individually or in combination. The concentration of the above-mentioned organic raw material to be used is not restricted. Yet, it is advantageous that the concentration is as high as possible within a range where the generation of succinic acid is not inhibited. The reaction is usually carried out in a concentration range of 5 to 30% (W/V), preferably 10 to 20% (W/V). Also, as the reaction progresses, the above-mentioned organic raw material decreases, and accordingly the organic raw material may be additionally supplemented.

The reaction solution containing the above-mentioned organic raw material is not restricted. For instance, it may be a medium for culturing a bacterium or a buffer such as a phosphate buffer. The reaction solution is preferably an aqueous solution containing a nitrogen source, an inorganic salt, and the like. Here, the nitrogen source is not restricted as long as the present bacterium can assimilate it to generate succinic acid. Specific examples thereof include a variety of organic or inorganic nitrogen compounds such as ammonium salts, nitric acid salts, urea, soybean hydrolysate, casein digest, peptone, yeast extract, meat extract and corn steep liquor. As the inorganic salt, various phosphate salts, sulfate salts and salts of metals such as magnesium, potassium, manganese, iron or zinc are used. Also, factors promoting growth including vitamins such as biotin, pantothenic acid, inositol or nicotinic acid, nucleotides and amino acids are supplemented as required. Also, it is preferred that, an appropriate amount of a commercially available antifoaming agent be added to the culture solution in order to inhibit foaming during the reaction.

It is preferred to allow the reaction solution to contain, for example, a carbonate ion, a bicarbonate ion, or carbon dioxide gas, in addition to the above-mentioned organic raw materials, nitrogen sources, inorganic salts and the like. The carbonate ion or the bicarbonate ion can be provided from magnesium carbonate, sodium carbonate, sodium bicarbonate, potassium carbonate and potassium bicarbonate, which can also be used as a neutralizer. As required, it can be provided from carbonic acid or bicarbonic acid, or salts thereof, or carbon dioxide gas. Specific examples of the salts of carbonic acid or bicarbonic acid include magnesium carbonate, ammonium carbonate, sodium carbonate, potassium carbonate, ammonium bicarbonate, sodium bicarbonate and potassium bicarbonate. And the carbonate ion or the bicarbonate ion is supplemented at a concentration of 1 to 500 mM, preferably 2 to 300 mM, further preferably 3 to 200 mM. In cases where the carbon dioxide gas is included, 50 mg to 25 g, preferably 100 mg to 15 g, further preferably 150 mg to 10 g of carbon dioxide gas is included per 1 L of the solution.

The pH of the reaction solution can be adjusted by adding sodium carbonate, sodium bicarbonate, potassium carbonate, potassium bicarbonate, magnesium carbonate, sodium hydroxide, calcium hydroxide, magnesium hydroxide or the like. The pH of the present reaction is usually a pH of 5 to 10, preferably a pH of 6 to 9.5. Hence, even during the reaction, the pH of the reaction solution is, as required, adjusted within the above-mentioned range using an alkaline substance, carbonic acid salts, urea or the like

The culture to obtain bacterial cells by growing the bacterium used in the present reaction is usually carried out in a range of 25°C to 35°C, which is the optimal growth temperature of a coryneform bacterium, more preferably a range of 25°C to 30°C, in particular preferably at about 30°C. As long as a prescribed amount of the bacterial cells can be obtained, any culturing time may be employed. And, it is usually 6 to 96 hours. The optimal growth temperature refers to a temperature at which the fastest growth rate can be attained under a condition used for the production of succinic acid.

Meanwhile, the production reaction of succinic acid is carried out at a temperature which is 2 to 20°C, preferably 7 to 15°C, higher than the optimal growth temperature of a coryneform bacterium used in the present reaction. Specifically, it is carried out within a range of 39 to 43°C in particular preferably 39 to 41°C. Time for the production reaction of succinic acid is preferably 1 hour to 168 hours, more preferably 3 hours to 72 hours.

The amount of the bacterial cells to be used in the reaction is not restricted, and 1 to 700 g/L, preferably 10 to 500 g/L, further preferably 20 to 400 g/L of cells is used.

During the seed culture of the bacterium, it is required to aerate and to stir in order to supply oxygen. On the other hand, the production reaction of succinic acid such as succinic acid may be carried out while aerating and stirring, or may be carried out under anaerobic atmosphere without aerating and supplying oxygen. The anaerobic atmosphere described herein can be achieved by a method such as allowing the reaction to take place in a sealed vessel with no aeration; allowing the reaction to take place while providing an inert gas such as nitrogen gas; aerating inert gases containing carbon dioxide gas.

By the bacterial reaction described above, succinic acid is generated and accumulated in the reaction solution. The succinic acid accumulated in the reaction solution (culture medium) is collected from the reaction solution by a conventional method. Specifically, solids such as bacterial cells are removed from the solution by centrifugation, filtration or the like; and then desalted with ion exchange resin or the like. Succinic acid can be collected from the resulting solution by purification by crystallization or column chromatography, or the like.

Further, in the present invention, after producing succinic acid by the above-mentioned method according to the present invention, the succinic acid-containing polymer can be produced by carrying out a polymerization reaction using the obtained succinic acid as a raw material. Recently, as the number of the environmentally conscious industrial products increases, polymer using raw materials of plant origin have drawn attention. In particular, the succinic acid produced in the present invention can be processed into polymers such as polyester and polyamide to be used. Specific examples of the succinic acid-containing polymer include succinic acid polyester obtained by polymerizing diols such as butanediol and ethylene glycol with succinic acid; and succinic acid polyamide obtained by polymerizing diamines such as hexamethylenediamine with succinic acid.

In addition, the succinic acid obtained by the production method according to the present invention or compounds containing the succinic acid can be used in food additives, pharmaceuticals, cosmetics and the like.

### Examples

The present invention will now be described in detail below by way of examples thereof; however the present invention is not restricted to these examples.

### <Preparation of a strain in which pyruvate carboxylase (PC) is enhanced>

### (A) Extraction of the genomic DNA of the Brevibacterium flavum MJ233 strain

In 10 mL of the medium A [urea 2 g, (NH₄)₂SO₄ 7 g, KH₂PO₄ 0.5 g, K₂HPO₄ 0.5 g, MgSO₄·7H₂O 0.5 g, FeSO₄·7H₂O 6 mg, MnSO₄·4-5H₂O 6 mg, biotin 200 µg, thiamin 100 µg, yeast extract 1 g, casamino acids 1 g and glucose 20 g were dissolved in distilled water 1 L], the *Brevibacterium flavum* MJ-233 strain was grown to the late logarithmic phase and bacterial cells were collected by centrifugation (10000 g, 5 minutes). The obtained bacterial cells were suspended in 0.15 mL of 10 mM NaCl/20 mM Tris buffer (pH8.0)/1 mM EDTA·2Na solution containing lysozyme at a concentration of 10mg/mL. Then, proteinase K was added to the above-mentioned suspension so as to attain a final concentration of 100 µg/mL and the mixture was kept at 37°C for 1 hour. Sodium dodecyl sulfate was added to the mixture so as to attain a final concentration of 0.5% and the resulting mixture was kept at 50°C for 6 hours to lyse the bacterial cells. To this lysate, an equal amount of a phenol/chloroform solution was added and the mixture was gently shaken at room temperature for 10 minutes. Thereafter the entire amount of the mixture was centrifuged (5,000×g, for 20 minutes, 10 to 12°C) and the supernatant fraction was obtained. Sodium acetate was added to the obtained fraction so as to attain a concentration of 0.3 M and then a twice amount of ethanol was added and mixed. The precipitate collected by centrifugation (15,000×g, 2 minutes) was washed with 70% ethanol and air-dried. To the obtained DNA, 5 mL of 10 mM Tris buffer (pH7.5)-1 mM EDTA·2Na solution was added and the mixture was kept to be static at 4°C overnight, which was later used as a template DNA for PCR.

### (B) Construction of a plasmid for replacement of the PC promoter

A DNA fragment of the N terminal region of the pyruvate carboxylase gene derived from the *Brevibacterium flavum* MJ233 strain was obtained by PCR with the DNA prepared in the above-mentioned (A) as a template, using synthetic DNAs (SEQ ID NO: 1 and SEQ ID NO: 2) which were designed on the basis of the sequence of the corresponding gene of the *Corynebacterium glutamicum* ATCC13032 strain (GenBank Database Accession No.BA000036; Cgl0689) whose entire genome sequence had been reported. As the DNA of SEQ ID NO: 1, one whose 5' terminus was phosphorylated was used. A reaction solution composition: template DNA 1 µL, PfxDNA polymerase (manufactured by Invitrogen Corporation) 0.2 µL, 1x concentration appended buffer, 0.3 µM each primer, 1 mM MgSO₄, 0.25 µM dNTPs were mixed to a final volume of 20 µL. A reaction temperature condition: using DNA thermal cycler PTC-200 (manufactured by MJ Research), a cycle composed of 94°C for 20 seconds, 60°C for 20 seconds, and 72°C for 1 minute was repeated 35 times. However, the heat retention at 94°C in the first cycle was set to last for 1 minute 20 seconds and the heat retention at 72°C in the last cycle was set to last for 4 minutes. To confirm the amplified product, the product was separated by 0.75% agarose (SeaKem GTG agarose: manufactured by FMC BioProducts) gel electrophoresis, and then visualized by ethidium bromide staining. A fragment of about 0.9 kb was detected. The DNA fragment of interest was recovered from the gel using QIAQuick Gel Extraction Kit (manufactured by QIAGEN) and this extracted DNA was used as a PC gene N terminal fragment.

Meanwhile, a TZ4 promoter fragment derived from the *Brevibacterium flavum* MJ233 strain, which promoter exhibits constitutive high expression, was prepared by PCR with the plasmid pMJPC1 (JP 2005-95169 A) as a template, using synthetic DNAs shown in SEQ ID NO: 3 and SEQ ID NO: 4. As the DNA of SEQ ID NO: 4, one whose 5' terminus was phosphorylated was used. A reaction solution composition: template DNA 1 µL, PfxDNA polymerase (manufactured by Invitrogen Corporation) 0.2 µL, 1x concentration appended buffer, 0.3 µM each primer, 1 mM MgSO₄, 0.25 µM dNTPs were mixed to a final volume of 20 µL. A reaction temperature condition: using DNA thermal cycler PTC-200 (manufactured by MJ Research), a cycle composed of 94°C for 20 seconds, 60°C for 20 seconds, and 72°C for 30 seconds was repeated 25 times. However the heat retention at 94°C in the first cycle was set to last for 1 minute 20 seconds and the heat retention at 72°C in the last cycle was set to last for 3 minutes. To confirm the amplified product, the product was separated by 1.0% agarose (SeaKem GTG agarose: manufactured by FMC BioProducts) gel electrophoresis, and then visualized by ethidium bromide staining. A fragment of about 0.5 kb was detected. The DNA fragment of interest was recovered from the gel using QIAQuick Gel Extraction Kit (manufactured by QIAGEN) and this extracted DNA was used as a TZ4 promoter fragment.

The PC gene N terminal fragment and the TZ4 promoter fragment, both of which are prepared above, were mixed and ligated using Ligation Kit ver.2 (manufactured by Takara Shuzo Co., Ltd.). Thereafter, the resultant was digested with a restriction enzyme, *Pst* I and separated by 1.0% agarose (SeaKem GTG agarose: manufactured by FMC BioProducts) gel electrophoresis. A DNA fragment with about 1.0 kb was recovered using QIAQuick Gel Extraction Kit (manufactured by QIAGEN) and the extracted DNA was used as a TZ4 promoter::PC gene N terminal fragment. Further, this DNA fragment was mixed with a DNA prepared by digesting *E. coli* plasmid pHSG299 (manufactured by Takara Shuzo Co., Ltd.) with *Pst* I and ligated using Ligation Kit ver.2 (manufactured by Takara Shuzo Co., Ltd.). The obtained plasmid DNA was used to transform *E. coli* (DH5α strain) . The thus obtained recombinant *E. coli* was spread on the LB agar medium containing 50 µg/mL kanamycin and 50 µg/mL X-Gal. Clones which formed white colonies on the medium were subjected to liquid culture by a conventional method and a plasmid DNA was purified. When the obtained plasmid DNA was digested with the restriction enzyme *Pst* I, an inserted fragment with about 1.0 kb was observed. This plasmid was named pMJPC17.1.

A DNA fragment of the 5' upstream region of the pyruvate carboxylase gene derived from the *Brevibacterium flavum* MJ233 strain was obtained by PCR with the DNA prepared in Example 1(A) as a template, using synthetic DNAs (SEQ ID NO: 5 and SEQ ID NO: 6) which were designed on the basis of the sequence of the corresponding gene (GenBank Database Accession No.BA000036) of the *Corynebacterium glutamicum* ATCC13032 strain whose entire genome sequence had been reported. A reaction solution composition: template DNA 1 µL, PfxDNA polymerase (manufactured by Invitrogen Corporation) 0.2 µL, 1x concentration appended buffer, 0.3 µM each primer, 1 mM MgSO₄, 0.25 µM dNTPs were mixed to a final volume of 20 µL. A reaction temperature condition: using DNA thermal cycler PTC-200 (manufactured by MJ Research), a cycle composed of 94°C for 20 seconds, 60°C for 20 seconds, and 72°C for 30 seconds was repeated 35 times. The heat retention at 94°C in the first cycle was set to last for 1 minute 20 seconds and the heat retention at 72°C in the last cycle was set to last for 5 minutes. To confirm the amplified product, the product was separated by 1.0% agarose (SeaKem GTG agarose: manufactured by FMC BioProducts) gel electrophoresis, and then visualized by ethidium bromide staining. A fragment of about 0.7 kb was detected. The DNA fragment of interest was recovered from the gel using QIAQuick Gel Extraction Kit (manufactured by QIAGEN). The recovered DNA fragment was phosphorylated at its 5' terminus by the T4 Polynucleotide Kinase (manufactured by Takara Shuzo Co., Ltd.) and ligated into a *Sma* I site of an *E. coli* vector, pUC119 (manufactured by Takara Shuzo Co., Ltd.) using Ligation Kit ver.2 (manufactured by Takara Shuzo Co., Ltd.). *E. coli* (DH5α strain) was transformed with the obtained plasmid DNA. The thus obtained recombinant *E. coli* was spread on the LB agar medium containing 50 µg/mL ampicillin and 50 µg/mL X-Gal. Clones which formed white colonies on the medium were subjected to liquid culture by a conventional method and a plasmid DNA was purified. The obtained plasmid DNA was subjected to a PCR reaction using synthetic DNAs (SEQ ID NO: 7 and SEQ ID NO: 6) as primers. A reaction solution composition: the above-mentioned plasmid 1 ng, Ex-TaqDNA polymerase (manufactured by Takara Shuzo Co., Ltd.) 0.2 µL, 1x concentration appended buffer, 0.2 µM each primer, and 0.25 µM dNTPs were mixed to a final volume of 20 µL. A reaction temperature condition: using DNA thermal cycler PTC-200 (manufactured by MJ Research), a cycle composed of 94°C for 20 seconds, 60°C for 20 seconds, and 72°C for 50 seconds was repeated 20 times. The heat retention at 94°C in the first cycle was set to last for 1 minute 20 seconds and the heat retention at 72°C in the last cycle was set to last for 5 minutes. As a result of confirming the presence of an inserted DNA fragment in this manner, a plasmid which shows an amplified product of about 0.7 kb was selected. This plasmid was named pMJPC5.1.

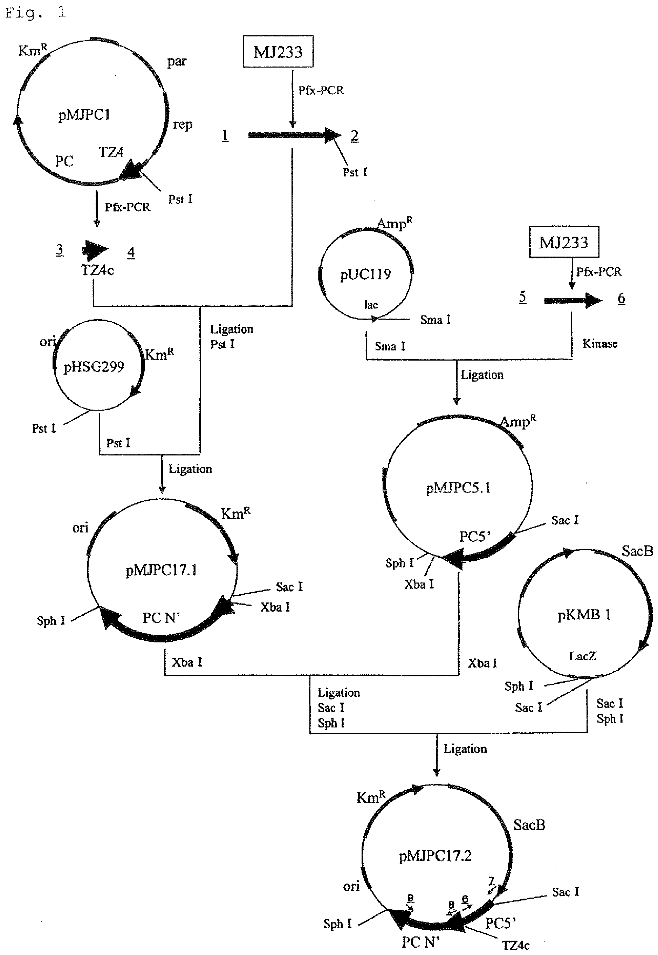

Subsequently, the above-mentioned pMJPC17.1 and pMJPC5.1 were individually digested with a restriction enzyme *Xba* I and then mixed, followed by ligation using Ligation Kit ver.2 (manufactured by Takara Shuzo Co., Ltd.) . This was digested with the restriction enzyme *Sac* I and the restriction enzyme *Sph* I to generate a DNA fragment, which was separated by 0.75% agarose (SeaKem GTG agarose: manufactured by FMC BioProducts) gel electrophoresis. The DNA fragment of about 1.75 kb was recovered using QIAQuick Gel Extraction Kit (manufactured by QIAGEN) . This DNA fragment in which the TZ4 promoter was inserted between the 5' upstream region and N terminal region of the PC gene and a DNA prepared by digesting plasmid pKMB1 harboring the *sacB* gene (JP 2005-95169 A) with *Sac* I and Sph I were mixed and ligated using Ligation Kit ver.2 (manufactured by Takara Shuzo Co., Ltd.). The obtained plasmid DNA was used to transform *E. coli* (DH5α strain) . The thus obtained recombinant *E. coli* was spread on the LB agar plate containing 50 µg/mL kanamycin and 50 µg/mL X-Gal. Clones which formed white colonies on the medium were subjected to liquid culture by a conventional method and a plasmid DNA was purified. By digesting the obtained plasmid DNA with the restriction enzymes *Sac* I and Sph I, an inserted fragment of about 1.75 kb was observed. This plasmid was named pMJPC17.2 (Figure 1).

### (C) Preparation of a strain in which PC is enhanced

A plasmid DNA used for transformation of the *Brevibacterium flavum* MJ233/ΔLDH (a strain with a decreased LDH activity: JP 2005-95169 A) was reprepared from the *E. coli* JM110 strain transformed with the plasmid DNA of pMJPC17.3 by the calcium chloride method (Journal of Molecular Biology, 53, 159, 1970). Transformation of the *Brevibacterium flavum* MJ233/ΔLDH strain was carried out by the electric pulse method (Res.Microbiol., Vol.144, p.181-185, 1993). The obtained transformant was spread on the LBG agar medium [tryptone 10 g, yeast extract 5 g, NaCl 5 g, glucose 20 g and agar 15 g were dissolved into distilled water 1 L] containing 25 µg/mL kanamycin. Since pMJPC17.2 is a plasmid incapable of replicating in the cells of the *Brevibacterium flavum* MJ233, in a strain grown on this medium, the kanamycin resistance gene and the *sacB* gene, which were originated from the plasmid, should be inserted on the genome as a result of homologous recombination between the pc gene on the plasmid and the same gene on the genome of the *Brevibacterium flavum* MJ233 strain. Next, the above-mentioned homologously recombinated strain was liquid-cultured in an LBG medium containing kanamycin 25 µg/mL. An aliquot of this culture solution corresponding to about one million bacterial cells was spread on the LBG medium containing 10% sucrose. As a result, several dozen of strains were obtained, which strains were thought to lose the *sacB* gene to be sucrose-insensitive by the second homologous recombination. The thus obtained strains include one in which the TZ4 promoter originated from pMJPC17.2 was inserted upstream of its PC gene and one which reverted to the wild type. Whether the PC gene is a promoter-replaced type or the wild type can be readily confirmed by directly subjecting bacterial cells obtained by liquid-culturing in the LBG medium to a PCR reaction and detecting the PC gene. When analysis using primers for PCR amplification of the TZ4 promoter and the PC gene (SEQ ID NO: 8 and SEQ ID NO: 9) is carried out, a DNA fragment of 678 bp should be observed in the promoter-substituted type. As a result of analyzing the bacterial strains which are rendered sucrose-insensitive by the above-mentioned method, a strain in which the TZ4 promoter was inserted was selected. The strain was named *Brevibacterium flavum* MJ233/PC-4/ΔLDH.

### (D) Measurement of the enzyme activity of pyruvate carboxylase

The obtained transformant in the above-mentioned (C), the *Brevibacterium flavum* MJ233/PC-4/ΔLDH strain, was cultured in 100 mL of the medium A containing glucose 2% and kanamycin 25 mg/L overnight. The obtained bacterial cells were collected and washed with 50 mM potassium phosphate buffer (pH7.5) 50 mL, and then resuspended in a buffer (20mL) with the same composition. The suspension was sonicated with SONIFIER 350 (manufactured by BRANSON) and centrifuged to obtain the supernatant, which was used as a cell-free extract. Using the obtained cell-free extract, the pyruvate carboxylase activity was measured. The measurement of the enzyme activity was carried out by allowing the enzyme to react at 25°C in a reaction solution containing 100 mM Tris/HCl buffer (pH7.5), 0.1 mg/10 ml biotin, 5 mM magnesium chloride, 50 mM sodium bicarbonate, 5 mM sodium pyruvate, 5 mM sodium adenosine triphosphate, 0.32 mM NADH, 20 units/1.5 ml malate dehydrogenase (manufactured by WAKO, originated from yeast) and the enzyme. 1 U was defined as an amount of enzyme which catalyzes a decrease of NADH by 1 µmol per 1 minute. The specific activity in the cell-free extract in which expression of pyruvate carboxylase was enhanced was 0.1 U/mg of protein. In contrast, in bacterial cells obtained by culturing the parent strain, the MJ233/ΔLDH strain, the specific activity was below the limit of detection of the present activity measurement method.

The *Brevibacterium flavum* MJ233/PC-4/ΔLDH strain was, as a succinic acid producing bacterium, used for a culture to prepare bacterial cells and for a reaction to produce a succinic acid.

Also, as a control, the *Brevibacterium flavum* MJ233/ΔLDH strain was used.

### [Example 1]

### Evaluation in a jar fermenter-1

### <Seed culture>

A medium (100 ml) (urea: 4 g, ammonium sulfate: 14 g, potassium phosphate monobasic: 0.5 g, potassium phosphate dibasic: 0.5 g, magnesium sulfate heptahydrate: 0.5 g, ferrous sulfate heptahydrate: 20 mg, manganese sulfate hydrate: 20 mg, D-biotin: 200 µg, thiamin hydrochloride: 200 µg, yeast extract: 5 g, casamino acids: 5 g, and distilled water: 1000 mL) was poured into a 500 mL Erlenmeyer flask and was subjected to heat sterilization at 120°C for 20 minutes. This was allowed to cool to room temperature, and then a 50% glucose aqueous solution (4 mL) which was sterilized in advance was added thereinto. The *Brevibacterium flavum* MJ233/PC-4/ΔLDH strain constructed above or the MJ233/ΔLDH strain was inoculated and the seed culture was carried out at 30°C for 16 hours.

### <Main culture>

A medium (365mL) (ammonium sulfate: 1.0 g, potassium phosphate monobasic: 1.5 g, potassium phosphate dibasic 1.5 g, potassium chloride: 1.67 g, magnesium sulfate heptahydrate: 0.5 g, ferrous sulfate heptahydrate: 40 mg, manganese sulfate hydrate: 40 mg, D-biotin: 1.0 mg, thiamin hydrochloride: 1.0 mg, SOLULYS (manufactured by Roquette) 5.8 g, antifoaming agent (CE457: manufactured by NOF Corporation: 1.0 g and distilled water: 1000 mL) was poured into a 1 L fermenter and subjected to heat sterilization at 120°C for 20 minutes. This was allowed to cool to room temperature, and then a 72% glucose aqueous solution (20 mL) which was sterilized in advance was added thereinto. To this, 20 mL of the above-mentioned seed culture broth was added and the mixture was kept at 30°C. The main culture was started with the pH being maintained, using 9.3% ammonia aqueous, so as not to be below 7.5, with aeration (600 mL per minute) and stirring (920 rpm). A dissolved oxygen level dropped to virtually zero, and then started increasing again to reach 1 ppm. At that point, a 72% glucose aqueous solution (about 700 µL) which was sterilized in advance was added and then the dissolved oxygen level went down to zero again. Every time the dissolved oxygen level increased again, addition of the glucose aqueous solution was repeated by the above-mentioned method, and the culturing was continued up to 22.5 hours after the beginning of the culture.

### <succinic acid production culture>

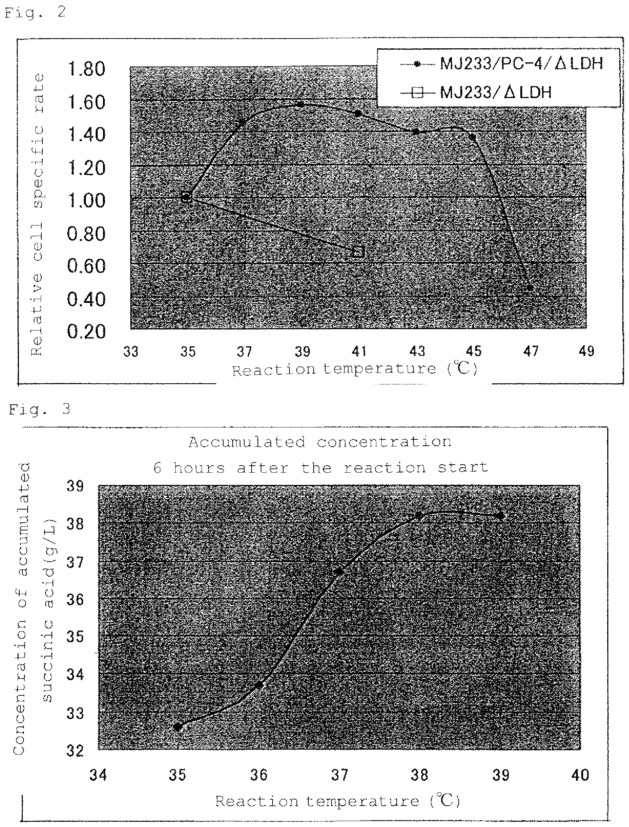

A medium (ammonium phosphate monobasic: 84.4 mg, ammonium phosphate dibasic: 75.8 mg, potassium chloride: 149.1 mg, magnesium sulfate heptahydrate: 0.2 g, ferrous sulfate heptahydrate: 8 mg, manganese sulfate hydrate: 8 mg, D-biotin: 80 µg, thiamin hydrochloride: 80 µg and distilled water: 200 mL) was poured into a 500 mL Erlenmeyer flask and subjected to heat sterilization at 120°C for 20 minutes. This was allowed to cool to room temperature and then poured into a 1 L jar fermenter (a plurality of these were prepared) . To this medium (200 mL), the culture broth obtained from the above-mentioned main culture (the MJ233/PC-4/ΔLDH strain or the MJ233/ΔLDH strain): 90 mL, a 50% glucose solution which was sterilized in advance: 100 mL and sterilized water: 120 mL were added and mixed. The mixtures containing the MJ233/PC-4/ΔLDH strain were kept at 35°C, 37°C, 39°C, 41°C, 43°C, 45°C and 47°C whereas the mixtures containing the MJ233/ΔLDH strain were kept at 35°C and 41°C. While maintaining the pH at 7.6 using an aqueous solution of ammonium bicarbonate: 89.8 g, 28% ammonia aqueous solution: 387 g and distilled water: 520 g; and stirring at 200 rpm, the succinic acid production reaction was carried out. Table 1 shows the relative cell specific rate under each temperature condition when the cell specific rate in the reaction of each bacterial strain at 35°C was set to 1.00. Also, relationships between the reaction temperature and the relative cell specific rate are shown in Figure 2. Here, the term "cell specific rate" represents an amount (g) of succinic acid which 1 g of bacterial cells produces per 1 hour.

**[Table 1]**

| Reaction temperature (°C) | Relative cell specific rate | |
|---|---|---|
| | MJ233/PC-4/ΔLDH | MJ233/ΔLDH |
| 35 | 1.00 | 1.00 |
| 37 | 1.46 | ND |
| 39 | 1.56 | ND |
| 41 | 1.51 | 0.66 |
| 43 | 1.39 | ND |
| 45 | 1.36 | ND |
| 47 | 0.44 | ND |

| | | |
|---|---|---|
| ND: no data | | |

From the results of Table 1 and Figure 2, it has been proven that the MJ233/PC-4/ΔLDH strain, unlike the MJ233/ΔLDH strain, exhibits a higher cell specific rate when allowed to react at 41°C than when allowed to react at 35°C. That is, it has been proven that the MJ233/PC-4/ΔLDH strain shows a higher efficiency of the succinic acid production when allowed to react at a high temperature of 37°C to 45°C. Also, in the MJ233/PC-4/ΔLDH strain, the efficiency of the succinic acid production was highest when the reaction temperature was between 39°C and 41°C.

### [Example 2]

### Evaluation in a jar fermenter-2

Using the *Brevibacterium flavum* MJ233/PC-4/ΔLDH strain obtained in the same manner as the seed culture of Example 1, evaluation of succinic acid production culture was carried out for temperature conditions at 35°C, 36°C, 37°C, 38°C, and 39°C in the same manner as described in Example 1. A relationship between the concentration of accumulated succinic acid and the reaction temperature after 6 hours is shown in Figure 3.

From Figure 3, it has been proven that the efficiency of succinic acid production sharply increases at or above a temperature of 37°C.

### [Example 3]

### Simple evaluation test

A culture broth of the *Brevibacterium flavum* MJ233/PC-4/ΔLDH strain or the MJ233/ΔLDH strain obtained in the same manner as the seed culture of Example 1 was centrifuged (10000×g, 5 minutes) to collect bacterial cells. The collected cells were suspended in a bacterial cell suspension medium (magnesium sulfate heptahydrate: 1 g, ferrous sulfate heptahydrate: 40 mg, manganese sulfate hydrate: 40 mg, D-biotin: 400 µg, thiamin hydrochloride: 400 µg, ammonium phosphate monobasic: 0.8 g, ammonium phosphate dibasic: 0.8 g, potassium chloride: 0.3 g, ammonium sulfate 66 g, and distilled water: 1000 mL) such that absorbance at OD₆₆₀ was 20. A plurality of 4 ml reactors containing 0.5 ml of the above-mentioned bacterial cell suspension and 0.5 mL of a substrate solution (glucose: 40 g, ammonium bicarbonate: 63.2 g, and distilled water: 1000mL) were prepared and allowed to react at 35°C and 41°C under atmosphere containing 5 to 6% carbon dioxide gas.

The concentration of accumulated succinic acid after 6 hours is shown in Table 2.

**[Table2]**

| Reaction temperature (°C) | Concentration of accumulated succinic acid after 6 hours (g/L) | |
|---|---|---|
| | MJ233/ΔLDH | MJ233/PC-4/ΔLDH |
| 35 | 3.5 | 7.8 |
| 41 | 2.7 | 9.4 |

From the Table 2, also in the simple evaluation test on a small scale, it has been proven that the MJ233/PC-4/ΔLDH strain has a higher efficiency of succinic acid production when allowed to react at a higher temperature.

Additionally, in the case of using a substrate solution containing sucrose as an organic raw material, similar results were also obtained.

### Industrial Applicability

By the method according to the present invention, succinic acid can be efficiently produced.

## Claims

1. A method for producing succinic acid, said method comprising:
allowing bacterial cells obtained by culturing a bacterium selected from *Brevibacterium flavum, Brevibacterium lactofermentum* and *Corynebacterium glutamicum,* or a treated product thereof selected from immobilized bacterial cells, a homogenate, centrifuged supernatant thereof, or a fraction, to act on an organic raw material in a reaction solution containing said organic raw material to thereby generate succinic acid, and
collecting said succinic acid;
wherein said bacterium has been modified such that pyruvate carboxylase activity is increased by 1.5 times or more per unit weight of bacterial cells, as compared with an unmodified strain, and said bacterial cells obtained by culturing said bacterium or said treated product thereof are allowed to act on said organic raw material at 39 to 43°C.

2. The method according to claim 1, wherein said bacterium has been further modified such that lactate dehydrogenase activity is decreased as compared with an unmodified strain.

3. The method according to claim 1 or 2, wherein said organic raw material is glucose or sucrose.

4. The method according to any one of claims 1 to 3, wherein said reaction solution contains a carbonate ion, a bicarbonate ion, or carbon dioxide gas.

5. The method according to any one of claims 1 to 4, wherein said organic raw material is allowed to act under anaerobic atmosphere.

6. A method for producing a succinic acid-containing polymer, comprising:
a step of producing succinic acid by the method according to any one of claims 1 to 5; and
a step of carrying out a polymerization reaction using said succinic acid obtained in the previous step as a raw material.

## Patentansprüche

1. Verfahren zur Produktion von Bernsteinsäure, wobei das Verfahren folgendes umfasst:
Es bakteriellen Zellen, erhalten durch Kultur eines Bakteriums, ausgewählt aus *Brevibacterium flavum, Brevibacterium lactofermentum* und *Corynebacterium glutamicum,* oder einem behandelten Produkt davon, ausgewählt aus immobilisierten bakteriellen Zellen, einem Homogenat, einem zentrifugierten Überstand davon, oder einer Fraktion davon, zu ermöglichen, auf ein organisches Rohmaterial in einer Reaktionslösung, welche das organische Rohmaterial enthält, einzuwirken, und
dadurch Bernsteinsäure zu generieren, und
die Bernsteinsäure zu gewinnen;
wobei das Bakterium so modifiziert wurde, dass Pyruvatcarboxylase-Aktivität um 1,5-fach oder mehr pro Einheitsgewicht (unit weight) der bakteriellen Zellen erhöht ist, verglichen mit einem nicht-modifizierten Stamm, wobei es den bakteriellen Zellen, erhalten durch Kultivieren von dem Bakterium oder dem behandelten Produkt davon, ermöglicht wird auf das organische Rohmaterial bei 39 bis 43 °C einzuwirken.

2. Verfahren gemäß Anspruch 1, wobei das Bakterium weiter modifiziert wurde, so dass Laktatdehydrogenase-Aktivität im Vergleich zu einem nicht-modifizierten Stamm verringert ist.

3. Verfahren gemäß Anspruch 1 oder 2, wobei das organische Rohmaterial Glukose oder Saccharose ist.

4. Verfahren gemäß irgendeinem der Ansprüche 1 bis 3, wobei die Reaktionslösung ein Carbonat-Ion, ein Bicarbonat-Ion oder ein Kohlendioxid-Gas enthält.

5. Verfahren gemäß irgendeinem der Ansprüche 1 bis 4, wobei es dem organischen Rohmaterial ermöglicht wird, unter anaerober Atmosphäre zu wirken.

6. Verfahren zur Produktion eines Bernsteinsäureenthaltenden Polymers, umfassend:
Einen Schritt der Produktion von Bernsteinsäure mittels des Verfahrens gemäß irgendeinem der Ansprüche 1 bis 5; und
einen Schritt des Ausführens einer Polymerisationsreaktion unter Verwendung der im vorhergehenden Schritt erhaltenen Bernsteinsäure als ein Rohmaterial.

## Revendications

1. Procédé de production d'acide succinique, ledit procédé comprenant :
permettre à des cellules bactériennes obtenues par une culture d'une bactérie choisie à partir des *Brevibacterium flavum, Brevibacterium lactofermentum* et *Corynebacterium glutamicum,* ou un produit traité de celle-ci choisi à partir de cellules bactériennes immobilisées, d'un homogénat, d'un surnageant centrifugé de celle-ci, ou d'une fraction, d'agir sur une matière première organique dans une solution réactionnelle contenant ladite matière première organique pour ainsi produire l'acide succinique, et
recueillir ledit acide succinique ;
dans lequel ladite bactérie a été modifiée de sorte que l'activité de la pyruvate carboxylase soit augmentée de 1,5 fois ou plus par unité pondérale de cellules bactériennes, par comparaison à une souche non modifiée, et lesdites cellules bactériennes obtenues par une culture de ladite bactérie ou dudit produit traité de celle-ci agissent sur ladite matière première organique à 39 jusqu'à 43° C.

2. Procédé selon la revendication 1, dans lequel ladite bactérie a été en outre modifiée de sorte que l'activité de la lactate dehydrogénase soit diminuée par comparaison avec une souche non modifiée.

3. Procédé selon la revendication 1 ou 2, dans lequel ladite matière première organique est le glucose ou le saccharose.

4. Procédé selon l'une quelconque des revendications 1 à 3, dans lequel ladite solution réactionnelle contient un ion carbonate, un ion bicarbonate, ou un gaz de dioxyde de carbone.

5. Procédé selon l'une quelconque des revendications 1 à 4, dans lequel ladite matière première organique agit sous une atmosphère anaérobie.

6. Procédé de production d'un polymère contenant de l'acide succinique, comprenant :
une étape de production d'acide succinique par le procédé selon l'une quelconque des revendications 1 à 5 ; et
une étape de mise en oeuvre d'une réaction de polymérisation utilisant ledit acide succinique obtenu à l'étape précédente, en tant que matière première.


### SEQUENCE LISTING

<110> Mitsubishi Chemical Corporation
<120> Method for production of succinic acid
<130>A8053-C8260
<150> JP2007-217277
   <151> 2007-08-23
<160> 9
<170> PatentIn version 3.3
<210> 1
   <211> 24
   <212> DNA
   <213> Artificial sequence
<220>
   <223> primer
<400> 1
   acgaagtgac tgctatcacc cttg 24
<210> 2
   <211> 23
   <212> DNA
   <213> Artificial sequence
<220>
   <223> primer
<400> 2
   cagaacttta ctgcatccgc aca 23
<210> 3
   <211> 36
   <212> DNA
   <213> Artificial sequence
<220>
   <223> primer
<400> 3
   gtggatgaga caggactatc tagagctaca gtgaca 36
<210> 4
   <211> 33
   <212> DNA
   <213> Artificial sequence
<220>
   <223> primer
<400> 4
   agaattgatc ttaggtcact aaaactaatt cag 33
<210> 5
   <211> 25
   <212> DNA
   <213> Artificial sequence
<220>
   <223> primer
<400> 5
   gtaggtatca cccatgcaca agttg 25
<210> 6
   <211> 23
   <212> DNA
   <213> Artificial sequence
<220>
   <223> primer
<400> 6
   cctagtatcg taacccccga ttc 23
<210> 7
   <211> 21
   <212> DNA
   <213> Artificial sequence
<220>
   <223> primer
<400> 7
   gttttcccag tcacgacgtt g 21
<210> 8
   <211> 24
   <212> DNA
   <213> Artificial sequence
<220>
   <223> primer
<400> 8
   actggcattg atgtcgatcc agca 24
<210> 9
   <211> 23
   <212> DNA
   <213> Artificial sequence
<220>
   <223> primer
<400> 9
   ctgttgccaa tttgcgaagc tca 23